# EUROPEAN PATENT SPECIFICATION

(11) **EP 3 744 838 B1**
(45) Date of publication and mention of the grant of the patent: **08.07.2026**
(21) Application number: 19177355.5
(22) Date of filing: 29.05.2019
(51) Int. Cl.: C12N 11/08, C12N 9/20, C07K 17/08, C12P 7/6458

(54) **LIPOLYTIC POLYMER PARTICLES FOR ESTERIFICATION AND INTERESTERIFICATION**
LIPOLYTISCHE POLYMERPARTIKEL ZUR VERESTERUNG UND UMESTERUNG
PARTICULES POLYMÈRES LIPOLYTIQUES POUR L'ESTÉRIFICATION ET L'INTERESTÉRIFICATION

(43) Date of publication of application: 02.12.2020
(73) Proprietor: Novozymes A/S, 2880 Bagsvaerd (DK)
(72) Inventor: ANDRIC, Pavle, 2880 Bagsvaerd (DK); NIELSEN, Per, Munk,, 2880 Bagsvaerd (DK); HOLM, Hans, Christian, 2880 Bagsvaerd (DK)
(74) Representative: NVS EPO Representatives

(56) References cited:
- WO-A1-2009/010561
- WO-A1-95/22606

## Description

### FIELD OF THE INVENTION

The present invention relates to small polymer particles for use in an enzymatic esterification or interesterification process, which have advantageous properties in both reaction rate and subsequent separation processes.

### BACKGROUND

Immobilization of lipolytic enzymes has been known for many years. An immobilized enzyme product may be used in enzymatic modification of an organic compound such as in organic synthesis processes, vegetable oil interesterification, biodiesel production etc.

Enzyme immobilization is the attachment of an enzyme protein on a carrier on which the enzyme is fixed, yet functional, where the enzyme is not released into the liquid (washed out) to which it is contacted. The most commonly immobilized enzymes are glucose isomerase used for isomerization reactions, and lipase used for, e.g., interesterification of vegetable oils and organic synthesis.

The industrial use of enzymes is often limited by their high cost and rapid inactivation. To improve their economic feasibility in industrial processes, enzymes are generally immobilized onto a particle. Immobilization facilitates re-use of the enzymes and may affect the selectivity and stability of the enzyme. Immobilization research has mainly focused upon means to enhance the transfer of enzymes onto the support and means to ensure that the enzymes remain active after being immobilized.

For use in non-aqueous solutions, lipolytic enzymes, such as lipases, can be immobilized on a number of different porous, inorganic carriers by absorption of an aqueous solution of lipase into the pore volume of the carrier, or by adsorption to the surface of the carrier, or by a combination of both adsorption and absorption followed by water removal by drying.

JP 5-292965A discloses an immobilized lipase and a method for preparing it.

WO 95/22606 (Pedersen et al.) describes an immobilization process based on a granulation process.

WO 99/33964 (Christensen et al.) describes an immobilization process wherein the enzyme is applied to a particulate porous carrier.

WO 2009/010561 discloses immobilization of a lipolytic enzyme on a preformed carrier particle, where the addition of a soluble polyol such as a carbohydrate or sugar alcohol significantly improves the performance of the immobilized lipolytic enzyme product with regard to initial enzyme activity, enzyme stability (half-life) and/or enzyme productivity.

Immobilized enzymes are known to be used in both continuous and batch enzymatic reactions within a variety of industrial applications, including waste water treatment, production of pharmaceuticals, high fructose corn syrup production, vegetable oil processing and synthesis of chemicals.

### SUMMARY OF THE INVENTION

In a first aspect, the present invention provides (a plurality of) enzyme particles, comprising 1-50% w/w of a lipolytic enzyme, 10-80% w/w of a hydrophobic polymer, 10-80% w/w of an organic filter aid, and 2-50% w/w of a water-soluble polyol selected from carbohydrates and sugar alcohols; wherein the particles have an average diameter below 100 µm.

In another aspect of the invention is provided methods for enzymatic esterification and interesterification, comprising contacting mixtures of free fatty acid/alcohol or triglycerides with the enzyme particles of the invention.

Other aspects and embodiments of the invention are apparent from the description and examples.

### DETAILED DESCRIPTION

The use of (lipolytic) enzyme catalyzed interesterification of fats/oils is well established. The reaction takes place in columns with a height of 1-5 meters filled with immobilized enzyme with a typical particle size of 300-1200 µm, where the oil is pumped through a set of columns with the total holding time required to achieve a certain conversion.

This type of setup (fixed bed column) is dependent upon an appropriate particle size of the enzyme to limit the pressure drop in the column. Another limitation for this kind of process is the mass transfer of oil into the particle. The easiest part of the enzyme activity to get access to is located at the surface, or in the near proximity to the particle surface. Therefore, the particle size for the process has until now been a compromise between having a size not too small to prevent high pressure drop, but still as small as possible to get a large surface area per kg of enzyme product. When using a large and robust particle size, it is necessary to take into account how much oil is held in the space between particles (voidage) as this oil will be mixed into the next batch of oil processed. This amount of oil can be so large that it in practice limits the use of this technology to productions with large volumes of same recipe to prevent mixing with oil from a previous batch.

With this invention, we have found a way to operate interesterification of oils/fats with a very small particle size (very high surface area), which results in an exceptionally high enzymatic activity. As explained above, this means that a lower amount of enzyme is needed because it is more accessible, and further that a lower amount of oil is trapped between the particles.

By the invented formulation of the particle it has been secured that the immobilized enzyme can be used either in batch/tank operation and filtered off after the reaction in standard oil filters, or it can be used in a fixed bed operation with a thin layer of enzyme such as 2-5 cm. The low dosage, low oil entrapment and possibility of re-using the enzyme provides a significantly improvement in cost-in-use compared to present technology.

Another advantage of the present invention is the use of hydrophobic polymers. The ability of lipolytic enzymes (such as lipases) - contrary to most other enzymes - to become adsorbed, stabilized and even activated on hydrophobic surfaces makes immobilization on hydrophobic supports advantageous.

The hydrophobic polymer surface may also contribute to the removal of hydrophilic reaction products, like glycerol, from the local environment of the lipolytic enzyme. In a biodiesel system, the formed glycerol (triglycerides + methanol → fatty acid methanol esters + glycerol) will stick to a silica carrier and "hide" the enzyme from the process - glycerol does not stick to the same degree on a hydrophobic carrier.

Unless otherwise indicated, all percentages are indicated as percent by weight (% w/w) throughout the application.

### Enzyme particles

The particles of the invention comprise 1-50% w/w of an immobilized lipolytic enzyme, 10-80% w/w of a hydrophobic polymer, 10-80% w/w of an organic filter aid, and 2-50% w/w of a water-soluble polyol selected from carbohydrates and sugar alcohols. The particles may be encapsulated in oil or fat; e.g. to form an oily powder or a slurry/suspension.

The particles have a volume-based particle size (D₅₀) below 100 µm, preferably 1-60 µm, more preferably 2-40 µm, and particularly 5-30 µm. The particle size is measured with a laser diffraction particle size analyzer.

The particles may be a homogenous mixture of the ingredients, i.e. the ingredients are uniformly distributed throughout the particles. Even if the individual particle is not uniform on a microscopic level, the ingredients may be randomly distributed with no overall structure, when a plurality of particles, such as at least 50 particles, is considered.

The particles are preferably porous. The pore volume may correspond to an oil uptake of at least 0.5 gram of oil per gram of particles, particularly at least 1 gram of oil per gram of particles. It may have a surface area of 5-1000 m²/g, 10-1000 m²/g, in particular 10-700 m²/g, more particularly 10-500 m²/g.

The particles may comprise the hydrophobic polymer, and the organic filter aid, in a total amount of 40-95% w/w, preferably 50-90% w/w.

In addition to the claimed ingredients, the particles may comprise inorganic, organic or both inorganic and organic material(s), which may be essentially insoluble in hydrophilic or hydrophobic liquids or mixtures thereof. The particles may further have a hydrophilic or hydrophobic surface. The particle surface can be modified and the enzyme may further be linked by hydrogen, ionic or covalent bonds or covalently cross-linked by, for example, glutaraldehyde treatment.

The particles may be prepared by spray drying a liquid (aqueous) mixture of the ingredients making up the particles (a hydrophobic polymer, an organic filter aid, enzyme and a water-soluble polyol selected from carbohydrates and sugar alcohols) or by absorption of the liquid solution of enzyme and a water-soluble polyol selected from carbohydrates and sugar alcohols, (separately or in a mixture) into a mixture of a hydrophobic polymer and an organic filter aid, followed by suitable drying technique (drying in a fluid bed, vacuum drier, etc.). The whole process could also be carried out in a combined/integrated mixer and dryer, such as a vacuum mixer. The mixture of filter aid and hydrophobic polymer (and any additional ingredients) may be preformed particles. By "preformed particles" is meant particles having their final form and structure before adding the enzyme and polyol. Generally, the ingredients can be added simultaneously or sequentially to optimize the production process.

The particles may contain less than 40% w/w water, preferably less than 25% w/w water, more preferably less than 10% w/w water, and most preferably less than 5% w/w water.

In order to make a final product with low dust properties and/or improved compatibility with the process in which it will be used (*e.g*., an interesterification process), the resulting particles can be sprayed with oil, or be blended with oil to obtain an oily powder or a slurry/suspension that encapsulates the particles in oil. The oil may be a plant derived oil, such as sunflower oil or another oil which is compatible with the process in which the particles will be used. If only a small amount of oil is used, the particles can be agglomerated into larger particles that can substantially reduce the amount of dust. The oil-encapsulated particles can also be dried subsequently.

The particles can also be sprayed or blended with fat to produce a solid block containing fat and small particles or processed through extrusion and pelletizing equipment to obtain large pellets, which may also include added fat as a 'vehicle'. Such particles can subsequently be coated with a preservation agent, for example a powderized preservation agent.

Dust is defined as particles with an aerodynamic diameter less than 50 µm. In aerosol science, it is generally accepted that particles with an aerodynamic diameter higher than 50 µm do not commonly remain airborne for very long. In this context, the aerodynamic diameter is defined as "the diameter of a hypothetical sphere of density 1 g/cm³ having the same terminal settling velocity in calm air as the particle in question, regardless of its geometric size, shape and true density." (WHO, 1997).

### Lipolytic enzyme

The enzyme to be immobilized according to the invention is a lipolytic enzyme, i.e. an enzyme which is capable of hydrolyzing carboxylic ester bonds to release carboxylate (EC 3.1.1). The lipolytic enzyme is an enzyme classified under the Enzyme Classification number E.C. 3.1.1.- (Carboxylic Ester Hydrolases) in accordance with the Recommendations (1992) of the International Union of Biochemistry and Molecular Biology (IUBMB). Thus, the lipolytic enzyme may exhibit hydrolytic activity, typically at a water/lipid interface, towards carboxylic ester bonds in substrates such as mono-, di- and triglycerides, phospholipids, thioesters, cholesterol esters, wax-esters, cutin, suberin, synthetic esters or other lipids mentioned in the context of E.C. 3.1.1. The lipolytic enzyme may, e.g., have triacylglycerol lipase activity (EC 3.1.1.3; 1,3-positionally specific or non-specific), phospholipase activity (A1 or A2; EC 3.1.1.32 or EC 3.1.1.4), esterase activity (EC 3.1.1.1) or cutinase activity (EC 3.1.1.74).

Suitable lipolytic enzymes (e.g. lipases) include those of bacterial or fungal origin. Chemically modified or protein engineered mutants are included. Examples include lipases from *Candida, C. Antarctica* (e.g. lipases A and B described in WO 88/02775), *C. rugosa* (*C. cylindracea), Rhizomucor, R. miehei, Hyphozyma, Humicola, Thermomyces, T. lanuginosus (H. lanuginosa* lipase) as described in EP 258 068 and EP 305 216, a *Pseudomonas* lipase, e.g. from *P. alcaligenes* or *P*. *pseudoalcaligenes* (EP 218 272), *P. cepacia* (EP 331 376), *P. glumae, P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B*. *pumilus* (WO 91/16422), lipase/phospholipase from *Fusarium oxysporum,* lipase from *F. heterosporum,* lysophospholipase from *Aspergillus foetidus,* phospholipase A1 from *A. oryzae,* lipase from *A. oryzae,* lipase/ferulic acid esterase from *A. niger,* lipase/ferulic acid esterase from *A. tubingensis,* lipase from *A. tubingensis,* lysophospholipase from *A. niger* and lipase from *F. solani.*

The lipase may be positionally site specific (*i.e.,* 1,3 specific) or non-specific, upon interaction with triglycerides as substrates.

Furthermore, a number of cloned lipases may be useful, including the *Penicillium camembertii* lipase described by Yamaguchi et al., (1991), Gene 103, 61-67), the *Geotricum candidum* lipase (Shimada, Y. et al., (1989), J. Biochem., 106, 383-388), and various *Rhizopus* lipases such as a *R. delemar* lipase (Hass, M.J et al., (1991), Gene 109, 117-113), a *R. niveus* lipase (Kugimiya et al., (1992), Biosci. Biotech. Biochem. 56, 716-719) and a *R. oryzae* lipase.

Other types of lipolytic enzymes such as cutinases may also be useful, e.g. cutinase from *Pseudomonas mendocina* (WO 88/09367), *Fusarium solani pisi* (WO 90/09446) or *H. insolens* (US 5,827,719).

The enzyme may be an enzyme variant produced, for example, by recombinant techniques. Examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Examples of commercially available lipases include Lipex^{™}, Lipoprime^{™}, Lipolase^{™}, Lipolase^{™} Ultra, Lipozyme^{™}, Palatase^{™}, Novozym^{™} 435, Quara^{™} and Lecitase^{™} (all available from Novozymes A/S). Other commercially available lipases include Lumafast^{™} (*Pseudomonas mendocina* lipase from Genencor International Inc.); Lipomax^{™} (*Ps*. *pseudoalcaligenes* lipase from DSM/Genencor Int. Inc.; and *Bacillus* sp. lipase from Genencor enzymes. Further lipases are available from other suppliers.

The enzyme may be added to the immobilization process in liquid form, such as an enzyme containing liquid (aqueous) medium.

The enzyme containing liquid medium is, in a particular embodiment of the present invention, a hydrophilic medium. In another particular embodiment, the liquid medium is aqueous. It may contain other organic or biological matter. Thus, it may be a fermentation broth or an enzyme concentrate solution obtainable by purifying a fermentation broth by e.g. ultrafiltration or by protein precipitation, separation and re-dissolution in another aqueous medium. It may further be substantially pure enzyme dissolved in an aqueous medium. In a special embodiment of the present invention the enzyme containing aqueous liquid has not been subjected to costly processing steps prior to immobilization to remove water such as evaporation nor has it been subjected to addition of non-aqueous solvents, e.g. organic solvents such as alcohols, e.g. (poly)ethylene glycol and/or (poly) propylene glycol.

The enzyme protein content of the enzyme particles is more than 1% w/w, but less than 50% w/w. In an embodiment, the enzyme protein content of the enzyme particles is more than 2% w/w, but less than 25% w/w. In a particular embodiment, the enzyme protein content of the enzyme particles is more than 4% w/w, but less than 20% w/w.

### Organic filter aid

Filter aids is a group of substantially inert materials that can be used in filtration pretreatment. An objective of adding filter aids is to improve the flow rate by decreasing cake compressibility and increasing cake permeability.

An organic filter aid, according to the invention, may be a cellulosic or lignocellulosic material. Preferably, the organic filter aid is substantially insoluble in both water and oil at standard ambient conditions (20°C). Thus, the organic filter aid may be an insoluble cellulose derivative.

In an embodiment, the organic filter aid is a wooden product (such as saw dust), or chemically derived from wood. Preferably, the organic filter aid is a water-insoluble polysaccharide, which may comprise beta(1→4) glycosidic bonds.

In a particularly preferred embodiment, the organic filter aid is cellulose (such as Filtracel from J.Rettenmaier & Sohne, Germany).

The organic filter aid may be mixed with other materials, as long as the mixture retains the overall properties of the organic filter aid and can be used as a filter aid in oils/fats.

The enzyme particles of the invention comprise the organic filter aid in an amount of 10-80% w/w, preferably 20-60% w/w.

### Hydrophobic polymers

The particles of the invention comprise hydrophobic polymers. Hydrophobic polymers are well-known in the art and provide a hydrophobic surface for adsorption of the lipolytic enzyme.

See for example the resins disclosed in WO 89/02916; or Veymar et al. "Evaluation of different commercial hydrophobic supports for the immobilization of lipases: tuning their stability, activity and specificity", RSC Adv., 2016, 6, 100281.

Preferred hydrophobic polymers are polymers of methacrylic acid and esters thereof, polymers of acrylic acid and esters thereof, polymers of styrene, polymers of divinylbenzene, and co-polymers thereof. The co-polymers may be random co-polymers or block co-polymers.

Polymers of methacrylic acid and esters thereof (also known as polymethacrylates) include, for example, poly(alkyl methacrylate), such as poly(methyl methacrylate), poly(ethyl methacrylate), poly(butyl acrylate), poly(octadecyl methacrylate), etc.

Polymers of acrylic acid and esters thereof (also known as polyacrylates) include, for example, poly(alkyl acrylate), such as poly(methyl acrylate), poly(ethyl acrylate), poly(butyl acrylate), poly(octadecyl acrylate), etc.

The hydrophobic polymers may further be cross-linked with, for example, divinylbenzene; or be functionalized with epoxy, amino, or alkyl groups. The degree of hydrophobicity of the hydrophobic polymers can be adjusted by appropriate selection of the monomers.

The hydrophobic polymers may be provided in the form of a macro-porous material, such as a macro-porous polymer matrix, or macro-porous polymer beads.

Such macro-porous materials may have a hydrophobic polymer content of at least 80% w/w, preferably at least 90% w/w, and more preferably at least 95% w/w. Most preferably, the material consists of hydrophobic polymers. The material may have an average particle size in the range of 1-100 µm, such as 1-50 µm.

In a preferred embodiment, the hydrophobic polymers are provided as macro-porous particles consisting of hydrophobic polymers with an average particle size in the range of 1-50 µm. Commercial products of such materials include resins from Purolite Corporation, and Lewatit resins from Lanxess.

The enzyme particles of the invention comprise the hydrophobic polymers in an amount of 10-80% w/w, preferably 20-60% w/w.

### Water-soluble polyol

The soluble polyol used in the invention is a carbohydrate or a sugar alcohol, typically with a solubility of at least 0.1 g per 100 ml of water at ambient temperature (e.g. 20°C). The carbohydrate may consist of 1-20 monosaccharide units. This includes monosaccharides and oligosaccharides such as disaccharides, trisaccharides, maltodextrin and dextrin.

The monosaccharide may be a hexose, either a ketose or an aldose, such as glucose, mannose, galactose, fructose and combinations thereof. Disaccharides may include sucrose, maltose, trehalose, isomaltose, cellubiose, melibiose, primeverose, rutinose, gentiobiose and lactose and combinations thereof. The trisaccharide may be maltotriose, raffinose or a combination thereof.

The carbohydrate may be a starch hydrolysate produced by hydrolysis, e.g. enzymatic hydrolysis, for example with an average of 2-20 monomer glucose units, such as dextrin with DE 6-8 or maltodextrin with DE 20-23 of starch.

The sugar alcohol may be monomeric, e.g. sorbitol or arabitol.

In a particularly preferred embodiment, the polyol is maltodextrin having a DE between 6 and 52. Maltodextrins with a DE above 20 are often referred to as glucose sirup.

The amount of the polyol (carbohydrate or sugar alcohol) used in the particle of the invention is 2 to 50%, e.g. 2 to 30%, 5 to 25% or 7 to 25% by weight of the enzyme particle.

### Uses of the particles

Particles comprising immobilized lipolytic enzymes, according to the invention, have potential applications in a wide range of enzymatic employed processes such as in the production of pharmaceuticals, specialty commodity chemicals, and vegetable oil processing.

Immobilized enzymes prepared in the context of the invention may be used for hydrolysis, synthesis or modification of organic substances. The hydrolysis, synthesis or modification preferably takes place in a medium essentially devoid of free water.

Accordingly, the invention encompasses a process for enzymatic modification of an organic compound comprising contacting in a reaction medium said organic compound with an immobilized enzyme product according to the invention.

The immobilized enzyme of the present invention may be used for enzymatic modification of an organic compound comprising contacting in a reaction medium said organic compound with an immobilized enzyme produced by the process of the invention.

In a particular embodiment of the present invention the modification is an esterification reaction comprising contacting a first reactant which is a carboxylic acid and a second reactant which is an alcohol with an immobilized lipase of the invention. The carboxylic acid may be selected from but not limited to the group consisting of fatty acids, lactic acid, benzoic acid, acrylic acid and methacrylic acid and the alcohol may be selected from but not limited to the group consisting of methanol, ethanol, isopropanol, polyols such as glycerol, sorbitol, isosorbide, xylitol, glucosides such as ethyl and methyl glucosides, neopentyl alcohol and propylene glycol.

The modification may be a chiral resolvation including an enantioselective synthesis or hydrolysis of carboxylic acid ester or amides; an aldol condensation reaction between two aldehydes; or an epoxidation of olefinic groups by percarboxylic acid produced in situ by the immobilized enzyme.

The modification may be a polyesterification reaction wherein the organic compound to be modified is a hydroxycarboxylic acid or oligomers of such compound e.g. lactic acid or 3-hydroxypropanoic acid. Or the carboxylic acid is a dicarboxylic acid selected from the group consisting of adipic acid, succinic acid, fumaric acid, 2,5-furandicarboxylic acid, glucaric acid, terephthalic acid and isophthalic acid, and the second reactant is selected from the group consisting of polyols such as 1,4-butanediol, 1,6-hexanediol, glycerol, sorbitol, isosorbide, neopentyl alcohol, or propylene glycol.

In another particular embodiment the modification is a ring opening polymerization reaction comprising contacting a lactone with an immobilized lipase produced by the present process. Prepared polymers may be homo or hetero polymers.

The modification may be a transesterification reaction comprising contacting a first reactant which is a carboxylic acid ester and a second reactant which is an alcohol with an immobilized lipase produced by the present process.

The modification may be an interesterification reaction comprising contacting a first reactant which is a carboxylic acid ester and a second reactant which is a second carboxylic acid ester with an immobilized lipase produced by the present process. In a more particular embodiment the modification is an interesterification reaction comprising contacting a first reactant which is a polycarboxylic acid ester and a second reactant which is a second polycarboxylic acid ester, with an immobilized lipase of the invention.

By interesterification of two different fats/oils the change in fatty acid positions resulting from the interesterification will impact the melting profile of the oils/fat mixture. This is measured by NMR and expressed as the percentage of solid fat at a given temperature in the typical range 10°C - 40°C. Examples of components are coconut fat and palm stearine.

The carboxylic acid ester may be selected from but not limited to the group consisting of alkyl esters of fatty acids, lactic acid, glucaric acid, benzoic acid, acrylic acid, methacrylic acid and wherein the alkyl may be methyl, ethyl, butyl and the alcohol may be selected from the group consisting of but not limited to methanol, ethanol, isopropanol, polyols such as glycerol, alkyl glucosides, such as ethyl glucoside or methyl glucoside, sorbitol, silicone and silicone derivatives, isosorbide, neopentyl alcohol and propylene glycol.

The modification may be a hydrolysis or synthesis producing an enantiopure compound; an amidation reaction comprising contacting a first reactant which is a carboxylic acid and a second reactant which is an amine with an immobilized lipase of the invention.

In a particular embodiment, the modification is an epoxidation reaction comprising in situ production of epoxidation agent with an immobilized enzyme produced by the present process.

In an embodiment of the present invention an immobilized lipase enzyme is used for an esterification, transesterification or interesterification process in a medium essentially devoid of free water. The transesterification may be used for fatty acid substitution, comprising contacting a first reactant and a second reactant with said immobilized lipase by which a substitution reaction occurs.

The first reactant may be a fatty acid ester, preferably a triglyceride or a mixture of triglycerides.

The second reactant may be another fatty acid ester different from the first reactant, preferably a triglyceride or a mixture of triglycerides. Further the second reactant may be an alcohol or a free fatty acid.

The medium in this preferred embodiment of the invention comprises an organic solvent, or it may consist essentially of triglycerides.

Said use of the invention may be applied in production of food products e.g. margarine or cocoa-butter substitutes, for production of esters for e.g. cosmetics, biofuel, etc.

### Processes

The invention also provides a process for conducting a reaction catalyzed by the lipolytic enzyme particles of the invention, comprising:
a) preparing a reaction mixture comprising reactants for the reaction, and
b) contacting the reaction mixture with the immobilized lipolytic enzyme particles at conditions which are effective for conducting the reaction.

The contact may be done by passing the reaction mixture through a packed-bed column of the immobilized lipolytic enzyme, a continuously stirred tank reactor holding the immobilized lipolytic enzyme, a moving bed reactor where the movement of the packed bed of immobilized enzyme is either co-current or counter-current to the reaction mixture, in a batch reactor, optionally with stirring or in any other type of reactor or combination of reactor in which the desired reaction can be carried out.

The lipolytic enzyme may be a lipase, the reactants may comprise a fatty acyl donor and an alcohol, and the reaction may form a fatty acid alkyl ester.

The lipolytic enzyme may be a lipase, the reactants may comprise at least two triglycerides, and the reaction may form different triglycerides. Thus, the reaction may be carried out for a time sufficient to change the melting properties of the mixture of triglycerides.

When the reaction catalyzed by the enzyme particles of the invention is carried out in a (stirred) tank reactor, the enzyme particles may subsequently be separated or recovered from the reactants by way of filtration. After separation, the enzyme particles may be used again (recycled) in the process.

It has been found that a method to re-use the enzyme can be established by allowing the reaction to take place with the enzyme fixed in a filter cake in a filter system. The oil (triglycerides) can be passing the filter cake one or more times to achieve the desired degree of reaction. By operating the reaction in a filter system, it is possible to use already existing equipment and to increase the rate of reaction. The higher rate of reaction is achieved by adding a higher amount of enzyme particles to the filter and enable reuse of the enzyme particles. The deactivation of the enzyme particles, which happens over time, is compensated by adding a small amount of extra enzyme particles to the filter for every batch. This can be repeated until the maximum filter cake thickness is reached, and the filter is full, and/or the maximum pressure drop over the filter is reached.

The present invention is further described by the following examples.

### EXAMPLES

Chemicals were commercial products of at least reagent grade.

### EXAMPLE 1

### Producing immobilized CalB on small polymer particles

A hydrophobic polymeric carrier (Lewatit VP OC 1600 from Lanxess) was prepared by drying 5-6 kg of wet raw material in a GEA fluid bed to produce 2-2.5 kg of dried material. Over drying was avoided, to prevent the material from becoming highly electrostatic.

200 grams of this dried material was crushed in a lab mill and mixed with cellulose filter aid. A mixture of maltodextrin and a *Candida antarctica* lipase B (CaIB) concentrate was made and applied onto the solid dry mixture of small polymer/cellulose particles in a 10L Lödige mixer. The material was partially dried in a mixer at 40°C for 30 min.

Subsequently, the particles comprising lipase, maltodextrin, hydrophobic polymer, and cellulose were encapsulated/coated in sunflower oil in a 10L Lödige mixer.

### EXAMPLE 2

### Lipase esterification

A 100 g biodiesel sample containing 3.3% free fatty acids (FFA) was esterified using 0.2% of the CalB polymer particles from Example 1. The temperature of the reaction mixture was 43°C. The reactor with the reaction mixture was mounted with a stirrer (350 rpm) and a drying system, which also supplied the methanol required for reacting with the FFA to form fatty acid methyl esters (FAME) - and water as by-product.

The drying system consisted of an airtube inlet at the bottom of the reactor. The airtube was used to add methanol/nitrogen to the reactor. The outlet from the headspace of the reactor was fed to the bottom of a flask with methanol at 25°C and bubbled into the methanol. In the methanol flask the water that was evaporated from the reactor and into the methanol/nitrogen was condensed, and a dry methanol/nitrogen was drawn from the headspace of the methanol flask and re-circulated back in the reactor. Air flow was 200 mL air per minute. The reaction was followed by measuring the FFA during 12 hours of reaction. The results are shown in Table 1.

**Table 1. FAME polishing using 0.2% CalB particles.**

| Reaction time (hours) | %FFA as C16 |
|---|---|
| 0 | 3.35 |
| 1 | 3.13 |
| 2 | 2.32 |
| 3 | 1.94 |
| 4 | 1.51 |
| 5 | 1.14 |
| 6 | 0.93 |
| 7 | 0.53 |
| 8 | 0.46 |
| 9 | 0.39 |
| 10 | 0.30 |
| 11 | 0.25 |
| 12 | 0.21 |

As evidenced by the data in Table 1, the free fatty acids were continuously removed from the biodiesel sample, during the enzymatic esterification of free fatty acids with methanol to produce fatty acid methyl esters (FAME).

## Claims

1. A plurality of enzyme particles, wherein said particles comprise
1-50% w/w of a lipolytic enzyme,
10-80% w/w of a hydrophobic polymer,
10-80% w/w of an organic filter aid, and
2-50% w/w of a water-soluble polyol selected from carbohydrates and sugar alcohols;
wherein the particles have an average diameter below 100 µm.

2. The particles of claim 1, which comprise the hydrophobic polymer in an amount of 20-60% w/w.

3. The particles of claim 1 or 2, which comprise the organic filter aid in an amount of 20-60% w/w.

4. The particles of any of claims 1-3, which comprise the hydrophobic polymer, and the organic filter aid in a total amount of 40-95% w/w, preferably 50-90% w/w.

5. The particles of any of claims 1-4, which comprise the polyol in an amount of 5-25% w/w.

6. The particles of any of claims 1-5, which comprise the lipolytic enzyme in an amount of 2-25% w/w.

7. The particles of any of claims 1-6, wherein the hydrophobic polymer is selected from the group consisting of polymers of methacrylic acid and esters thereof, polymers of acrylic acid and esters thereof, polymers of styrene, polymers of divinylbenzene, and co-polymers thereof.

8. The particles of any of claims 1-7, wherein the organic filter aid is a water-insoluble polysaccharide, preferably comprising beta(1→4) glycosidic bonds.

9. The particles of any of claims 1-8, wherein the organic filter aid is cellulose.

10. The particles of any of claims 1-9, wherein the polyol is selected from the group consisting of dextrin, maltodextrin, trisaccharides, disaccharides, monosaccharides, and mixtures thereof.

11. The particles of any of claims 1-10, wherein the polyol is maltodextrin having a Dextrose Equivalent (DE) between
6 and 52.

12. The particles of any of claims 1-11, wherein the lipolytic enzyme is a lipase.

13. The particles of any of claims 1-12, in the form of a substantially homogenous composition of the ingredients, prepared by spray drying or absorption, followed by drying.

14. The particles of any of claims 1-13, which have an average diameter of 1-60 µm.

15. A powder or a slurry/suspension comprising the particles of any of claims 1-14 and at least 10% oil or fat.

16. A method for enzymatic interesterification, comprising contacting a mixture of triglycerides with the particles according to any of claims 1-14.

17. A method for enzymatic esterification, comprising contacting a mixture of free fatty acid and alcohol with the particles according to any of claims 1-14.

## Patentansprüche

1. Vielzahl von Enzympartikeln, wobei die Partikel
1-50% Gew./Gew. eines lipolytischen Enzyms,
10-80% Gew./Gew. eines hydrophoben Polymers,
10-80% Gew./Gew. eines organischen Filterhilfsmittels, und
2-50% Gew./Gew. eines wasserlöslichen Polyols, ausgewählt aus Kohlenhydraten und Zuckeralkoholen, umfassen;
wobei die Partikel einen mittleren Durchmesser unter 100 µm aufweisen.

2. Partikel nach Anspruch 1, die das hydrophobe Polymer in einer Menge von 20-60% Gew./Gew. umfassen.

3. Partikel nach Anspruch 1 oder 2, die das organische Filterhilfsmittel in einer Menge von 20-60% Gew./Gew. umfassen.

4. Partikel nach einem beliebigen der Ansprüche 1-3, die das hydrophobe Polymer und das organische Filterhilfsmittel in einer Gesamtmenge von 40-95 Gew.-%, vorzugsweise 50-90% Gew./Gew. umfassen.

5. Partikel nach einem beliebigen der Ansprüche 1-4, die das Polyol in einer Menge von 5-25% Gew./Gew. umfassen.

6. Partikel nach einem beliebigen der Ansprüche 1-5, die das lipolytische Enzym in einer Menge von 2-25% Gew./Gew. umfassen.

7. Partikel nach einem beliebigen der Ansprüche 1-6, wobei das hydrophobe Polymer aus der Gruppe ausgewählt ist, die aus Polymeren von Methacrylsäure und Estern davon, Polymeren von Acrylsäure und Estern davon, Polymeren von Styrol, Polymeren von Divinylbenzol, und Copolymeren davon besteht.

8. Partikel nach einem beliebigen der Ansprüche 1-7, wobei das organische Filterhilfsmittel ein wasserunlösliches Polysaccharid ist, das vorzugsweise beta(1→4)-glykosidische Bindungen umfasst.

9. Partikel nach einem beliebigen der Ansprüche 1-8, wobei das organische Filterhilfsmittel Cellulose ist.

10. Partikel nach einem beliebigen der Ansprüche 1-9, wobei das Polyol aus der Gruppe ausgewählt ist, die aus Dextrin, Maltodextrin, Trisacchariden, Disacchariden, Monosacchariden und Gemischen davon besteht.

11. Partikel nach einem beliebigen der Ansprüche 1-10, wobei das Polyol Maltodextrin mit einem Dextroseäquivalent (DE) zwischen 6 und 52 ist.

12. Partikel nach einem beliebigen der Ansprüche 1-11, wobei das lipolytische Enzym eine Lipase ist.

13. Partikel nach einem beliebigen der Ansprüche 1-12 in Form einer im Wesentlichen homogenen Zusammensetzung der Bestandteile, hergestellt durch Sprühtrocknen oder Absorption, gefolgt von Trocknen.

14. Partikel nach einem beliebigen der Ansprüche 1-13, die einen mittleren Durchmesser von 1-60 µm aufweisen.

15. Pulver oder Aufschlämmung/Suspension, umfassend die Partikel gemäß einem beliebigen der Ansprüche 1-14 und mindestens 10% Öl oder Fett.

16. Verfahren zur enzymatischen Umesterung, umfassend Inkontaktbringen eines Gemisches aus Triglyceriden mit den Partikeln gemäß einem beliebigen der Ansprüche 1-14.

17. Verfahren zur enzymatischen Veresterung, umfassend Inkontaktbringen eines Gemisches aus freier Fettsäure und Alkohol mit den Partikeln gemäß einem beliebigen der Ansprüche 1-14.

## Revendications

1. Pluralité de particules d'enzyme, dans laquelle lesdites particules comprennent
1 à 50 % en poids/poids d'une enzyme lipolytique,
10 à 80 % en poids/poids d'un polymère hydrophobe,
10 à 80 % en poids/poids d'un auxiliaire de filtration organique, et
2 à 50 % en poids/poids d'un polyol soluble dans l'eau choisi parmi les hydrates de carbone et les alcools de sucre ;
dans laquelle les particules ont un diamètre moyen inférieur à 100 µm.

2. Particules selon la revendication 1, qui comprennent le polymère hydrophobe en une quantité de 20 à 60 % en poids/poids.

3. Particules selon la revendication 1 ou 2, qui comprennent l'auxiliaire de filtration organique en une quantité de 20 à 60 % en poids/poids.

4. Particules selon l'une quelconque des revendications 1 à 3, qui comprennent le polymère hydrophobe et l'auxiliaire de filtration organique en une quantité totale de 40 à 95 % en poids/poids, de préférence de 50 à 90 % en poids/poids.

5. Particules selon l'une quelconque des revendications 1 à 4, qui comprennent le polyol en une quantité de 5 à 25 % en poids/poids.

6. Particules selon l'une quelconque des revendications 1 à 5, qui comprennent l'enzyme lipolytique en une quantité de 2 à 25 % en poids/poids.

7. Particules selon l'une quelconque des revendications 1 à 6, dans lesquelles le polymère hydrophobe est choisi dans le groupe constitué par les polymères d'acide méthacrylique et de ses esters, les polymères d'acide acrylique et de ses esters, les polymères de styrène, les polymères de divinylbenzène, et leurs copolymères.

8. Particules selon l'une quelconque des revendications 1 à 7, dans lesquelles l'auxiliaire de filtration organique est un polysaccharide insoluble dans l'eau, comprenant de préférence des liaisons bêta(1→4) glycosidiques.

9. Particules selon l'une quelconque des revendications 1 à 8, dans lesquelles l'auxiliaire de filtration organique est la cellulose.

10. Particules selon l'une quelconque des revendications 1 à 9, dans laquelle le polyol est choisi dans le groupe constitué par la dextrine, la maltodextrine, les trisaccharides, les disaccharides, les monosaccharides, et leurs mélanges.

11. Particules selon l'une quelconque des revendications 1 à 10, dans lesquelles le polyol est une maltodextrine ayant un équivalent dextrose (DE) compris entre 6 et 52.

12. Particules selon l'une quelconque des revendications 1 à 11, dans lesquelles l'enzyme lipolytique est une lipase.

13. Particules selon l'une quelconque des revendications 1 à 12, sous la forme d'une composition sensiblement homogène des ingrédients, préparées par séchage par pulvérisation ou adsorption, puis séchage.

14. Particules selon l'une quelconque des revendications 1 à 13, qui ont un diamètre moyen de 1 à 60 µm.

15. Poudre ou bouillie/suspension comprenant les particules de l'une quelconque des revendications 1 à 14 et au moins 10 % d'huile ou de graisse.

16. Méthode d'inter-estérification enzymatique, comprenant la mise en contact d'un mélange de triglycérides avec les particules de l'une quelconque des revendications 1 à 14.

17. Méthode d'estérification enzymatique, comprenant la mise en contact d'un mélange d'acide gras libre et d'alcool avec les particules de l'une quelconque des revendications 1 à 14.
